# EUROPÄISCHE PATENTSCHRIFT

(11) **EP 3 432 962 B1**
(45) Veröffentlichungstag und Bekanntmachung des Hinweises auf die Patenterteilung: **28.04.2021**
(21) Anmeldenummer: 17727077.4
(22) Anmeldetag: 21.03.2017
(51) Int. Cl.: A61M 16/00, A61B 5/087, G01F 1/34

(54) **ANORDNUNG ZUR MESSUNG DES VOLUMENSTROMES EINES PATIENTEN BEI DER MASCHINELLEN BEATMUNG**
ASSEMBLY FOR MEASURING THE FLOW RATE OF A PATIENT DURING MECHANICAL VENTILATION
SYSTÈME POUR MESURER LE DÉBIT VOLUMIQUE D'UN PATIENT SOUS VENTILATION ARTIFICIELLE

(30) Priorität: 23.03.2016 DE 102016105467
(43) Veröffentlichungstag der Anmeldung: 30.01.2019
(73) Patentinhaber: Fritz Stephan GmbH Medizintechnik, 56412 Gackenbach (DE)
(72) Erfinder: MAINUSCH, Georg, 56412 Gackenbach (DE); SCHLIECKER, Jan, 56412 Gackenbach (DE); SCHALLER, Peter, 01326 Dresden (DE)
(74) Vertreter: Kaufmann, Sigfrid
(86) Internationale Anmeldenummer: PCT/DE2017/100222
(87) Internationale Veröffentlichungsnummer: WO 2017/162238

(56) Entgegenhaltungen:
- WO-A1-2012/048936
- WO-A2-02/089920
- DE-A1- 19 617 432
- US-A1- 2006 117 856
- US-A1- 2011 270 541

## Beschreibung

Die Erfindung betrifft das Gebiet der Beatmungstechnik, insbesondere eine Anordnung zum Messen eines Gasflusses, d. h. eines Volumenstromes, im Rahmen einer maschinellen Beatmung eines Patienten.

Beatmungssysteme ermöglichen das maschinelle Beatmen von Patienten z. B. nach einer Operation oder frühgeborenen Kindern nach der Geburt. Das einem Patienten bei maschineller Beatmung zugeführte Atemgas wird in einem Atemgasbefeuchter angewärmt und befeuchtet. Es ist üblich, den vom Atemgasbefeuchter zum Patienten führenden Beatmungsschlauch als Inspirationsschlauch und den vom Patienten zum Beatmungsgerät führenden Beatmungsschlauch als Exspirationsschlauch zu bezeichnen. Inspirationsschlauch und Exspirationsschlauch bilden in Verbindung mit dem Y-Stück das Beatmungsschlauchsystem. Die Atemwege des Patienten werden über einen Beatmungstubus und einen Tubuskonnektor mit dem Y-Stück verbunden.

Die Messung des Volumenstromes des Atemgases bzw. der Atmung bei einem beatmeten Patienten erfolgt nach dem Stand der Technik nach anemometrischen Verfahren oder nach dem Differenzdruck-Verfahren.

Bei dem Differenzdruck-Verfahren durchströmt das Atemgas vom und zum Patienten einen sogenannten Pneumotachometer, d. h. ein pneumatisches Widerstandselement, zwischen dessen beiden Enden sich ein Differenzdruck ausbildet, der von einem Differenzdrucksensor gemessen wird. AU 2011201469 A1 beschreibt beispielsweise einen solchen Strömungswiderstand zur Messung eines Gasvolumenstromes bzw. einer im Gasstrom auftretenden Druckdifferenz. Auch in GB 2 446 124 A ist eine Vorrichtung zur Überwachung des Atemgasflusses während der Beatmung eines Patienten offenbart, wobei der Druckabfall an einem Strömungswiderstand als Messgröße zur Bestimmung des Atemgas-Volumenstroms herangezogen wird.

Wie in US 5 316 009 A beschrieben, wird dieser Pneumotachometer vorzugsweise zwischen dem Patientenanschluss des Y-Stückes und dem Tubuskonnektor angeordnet, da so der Volumenstrom des Atemgases vom und zum Patienten exakt messbar ist. Diese Anordnung besitzt andererseits den Nachteil, dass durch das Volumen des Pneumotachometers der technische Totraum erhöht wird, wodurch die Sauerstoffsättigung gemindert bzw. die CO₂-Abgabe bei der Exspiration behindert und die erforderliche Atemtiefe erhöht wird. Dies kann insbesondere für Säuglinge eine lebensbedrohliche Gefahr darstellen, da im Totraum die CO₂-Konzentration im Tubuskonnektor weit über das normale Maß ansteigen kann.

Um diesen Nachteil zu vermeiden, können, wie in DE 196 17 432 A1 vorgeschlagen, zwei pneumatische Widerstandselemente vorgesehen werden, wobei das eine pneumatische Widerstandselement in dem Inspirationszweig und das zweite pneumatische Widerstandselement in dem Exspirationszweig angeordnet werden. Der Volumenstrom zum Patienten bestimmt sich danach aus der Differenz der Volumenströme beider pneumatischer Widerstandselemente.

In grundsätzlich bekannter Weise befindet sich der Differenzdrucksensor in dem Beatmungsgerät. Die Verbindung zu dem pneumatischen Widerstandselement erfolgt über einen Zwillingsschlauch, dessen Länge im Bereich zwischen 100 und 150 cm liegt. Die Messung des Beatmungsdruckes erfolgt ebenfalls patientennah, wobei auch hier die Weiterleitung des anliegenden Druckes über einen Schlauch gleicher Länge zu einem im Beatmungsgerät angebrachten Drucksensor erfolgt. Nachteilig an diesen langen Druckmess-Schläuchen ist, dass hierdurch das Regelungsverhalten der üblicherweise im Beatmungsgerät implementierten Regelungsalgorithmen für Atemgas-Druck-sowie Volumenstrom verschlechtert wird.

Nachteilig am patientennah angeordneten Strömungswiderstand ist weiterhin, dass dieser durch möglicherweise in dem ausgeatmeten Atemgas vorhandene Keime kontaminiert wird, wobei auch eine Kontamination der über die Druckmess-Schläuche mit dem Strömungswiderstand verbundenen Sensoren möglich ist. Somit müssen die kontaminierten Bauteile im Falle einer Wiederverwendung einer aufwendigen Reinigung und Sterilisation unterzogen werden.

Zur Umgehung dieses Problem ist aus US 7 174 789 B2 eine Anordnung aus einem als Wegwerfteil konzipierten pneumatischen Widerstandselement und ein lösbar mit diesem verbindbares Druckmessteil bekannt, wobei Keimsperreinrichtungen eine Kontamination des wiederverwendbaren Druckmessteils verhindern. Allerdings weist auch diese Anordnung einen großen technischen Totraum auf.

Weiterhin bekannt aus US 2010/0106040 A1 sind ein System zum Erfassen des Atmungsdruck mit einem tragbaren, abnehmbaren Druckwandler, aus

DE 196 17 432 A1 eine Anordnung zur Messung des Volumenstromes der Atmung mit einem Differenzdrucksensor zwischen Inspirationszweig und Expirationszweig sowie aus US 5 925 831 A ein Strömungssensor zur Atemstrommessung mit aerodynamisch profilierten Leitschaufeln als Strömungswiderstand.

Der Erfindung liegt die Aufgabe zugrunde, die oben genannten Nachteile, wie eine verschlechterte Beatmungsregelung aufgrund zu langer Druckmessschläuche und einen großen technischen Totraum, zu vermeiden, wobei eine unkompliziert handzuhabende, insbesondere ohne Desinfektions- oder Sterilisationsschritt verwendbare Anordnung für eine Regelung und/oder Überwachung des Atemgasflusses insbesondere von maschinell beatmeten, frühgeborenen Säuglingen bereitgestellt werden soll.

Die Lösung dieser Aufgabe erfolgt gemäß den kennzeichnenden Merkmalen des Patentanspruchs 1; zweckmäßige Ausgestaltungen der Erfindung finden sich in den Unteransprüchen.

Gemäß der Erfindung wird eine Anordnung zur Messung eines Volumenstromes, d. h. eines Gasflusses, eines einem Patienten von einem Beatmungsgerät zur Beatmung zugeführten Atemgases und eines Beatmungsdruckes in einem an das Beatmungsgerät angeschlossenen Beatmungsschlauchsystem bereitgestellt, die ein Y-Stück mit zwei in das Y-Stück monolithisch integrierten pneumatischen Widerstandselementen aufweist.

Weiterhin weist die Anordnung ein lösbar mit dem Y-Stück verbindbares Elektronikmodul auf, wobei an jedem pneumatischen Widerstandselement des Y-Stücks ein Gasdruck in Strömungsrichtung vor und hinter demselben, insbesondere ein Differenzdruck, messbar ist. Die Sensoren für die Messung des jeweiligen Differenzdrucks und des Beatmungsdruckes sowie eine Auswerteeinheit zumindest für die Ausgangssignale der Sensoren, d. h. des Drucksensors und der Differenzdrucksensoren, sind in dem separaten Elektronikmodul untergebracht, wobei mittels der Auswerteeinheit der von dem beatmeten Patienten aufgenommene bzw. abgegebene Atemluftstrom aus den von den Differenzdrucksensoren ermittelten Differenzdrücken berechenbar ist. Ebenso ist von der Auswerteeinheit eine Bestimmung des patientennahen Drucks mittels Auslesens des von dem Drucksensor erfassten Drucks durchführbar.

Somit umfasst die Anordnung zwei lösbar miteinander verbindbare Bauteile - das Y-Stück und das Elektronikmodul, wobei das Y-Stück als Einweg-Einheit und das Elektronikmodul als Mehrweg-Einheit konzipiert sind. Der sonst übliche Verbindungsschlauch zwischen den beiden Bauteilen entfällt. Für eine Verbindung weist das Y-Stück einen Fluidmessausgang zur Messung des Beatmungsdruckes sowie für jeden seiner pneumatischen Widerstandselemente jeweils einen patientennahen und einen patientenfernen Fluidmessausgang, z. B. in Form eines Druckmessstutzens, auf, an die das Elektronikmodul angeschlossen wird. Das Elektronikmodul weist hierfür fünf Fluidmesseingänge auf.

Um eine Kontamination der Drucksensoren durch das (ggf. keimbelastete) Atemgas des Patienten auszuschließen, werden in den Fluidmessausgängen, d. h. den von dem Inspirationszweig bzw. Exspirationszweig des Y-Stücks abgehenden Strömungskanälen, antibakterielle Mittel, die eine solche Kontamination unterbinden, im Folgenden Keimsperreinrichtung genannt, vorgesehen. Dies können Bakterienfilter sein oder Mittel, die auf andere, dem Fachmann bekannte Weise, eine Weiterleitung von Keimen verhindern, wie zum Beispiel antibakterielle Beschichtungen der pneumatischen Verbindungsleitungen.

Somit kommt erfindungsgemäß nur das Y-Stück in direkten Kontakt mit ggf. im Atemgas des beatmeten Patienten vorhandenen Keimen, indem die Keimsperreinrichtung eine Kontamination des Elektronikmoduls verhindert.

Diese Keimsperreinrichtungen sind bevorzugt in oder an den mit dem Elektronikmodul zu verbindenden Strömungskanälen des Y-Stücks angeordnet, können aber auch nur oder zusätzlich in dem Elektronikmodul vorgesehen sein. Darüber hinaus kann das Gehäuse des Elektronikmoduls aus einem antibakteriellen Material bestehen oder antibakteriell beschichtet sein.

Das Y-Stück mit den beiden pneumatischen Widerstandselementen ist, wie bereits erwähnt, als Einweg-Gebrauchsartikel ausgelegt, wobei die pneumatische Verbindung zwischen den beiden Messanschlüssen eines jeden pneumatischen Widerstandselements und den Gasdruck-Messeingängen der im Elektronikmodul angeordneten Differenzdrucksensoren durch pneumatische Verbindungsleitungen in Form dünner Strömungskanäle gewährleistet ist. Der Drucksensor zur Messung des Beatmungsdrucks ist gleichermaßen (als separate Komponente) im Elektronikmodul angeordnet, wobei der Gasdruck-Messeingang des Drucksensors ebenfalls über einen dünnen Strömungskanal mit einem, vorzugsweise zentral am Y-Stück angeordneten, Fluidmessausgang verbindbar ist. Die in dem Elektronikmodul angeordneten pneumatischen Verbindungsleitungen sind hierbei als kurze Strömungskanäle, d. h. mit einer Länge von weniger als 60 mm, ausgelegt.

Das die pneumatischen Widerstandselemente beinhaltende Y-Stück ist über eine lösbare Verbindung derart mit dem Elektronikmodul verbindbar, dass es auf einfache Weise, d. h. lediglich durch Lösen der Verbindung, von dem Elektronikmodul getrennt und nach einmaligem Gebrauch entsorgt werden kann.

Das Y-Stück kann mittels einer formschlüssigen und/oder kraftschlüssigen Verbindung, beispielsweise vermittels eines Kopplungselements oder aufgrund entsprechender baulicher Gestaltung der Gehäuse von Y-Stück und Elektronikmodul, mit dem Elektronikmodul verbunden bzw. an dem Elektronikmodul befestigt sein.

Um ein unbeabsichtigtes Lösen der Verbindung zu vermeiden, kann eine Verriegelung vorgesehen sein, sodass nur nach Öffnen der Verriegelung das Y-Stück vom Elektronikmodul lösbar ist. Zum Beispiel ist die Verriegelung in Form einer oder mehrerer gefederten Nasen ausgeführt, die beim Verbinden der Teile in eine Nut einrasten.

Aufgrund der Lösbarkeit von Y-Stück und Elektronikmodul sind auch die pneumatischen Verbindungen zwischen den Fluidmessausgängen des Y-Stücks und den Messeingängen der im Elektronikmodul angeordneten Sensoren lösbar.

Die Fluidmessausgänge können als in das Gehäuse des Y-Stücks eingebrachte Fluid-Messausgangsöffnungen ausgeführt sein. Gleichermaßen kann das Gehäuse des Elektronikmoduls Fluid-Messeingangsöffnungen aufweisen, an die die Sensoren mittels der pneumatischen Verbindungsleitungen angeschlossen sind. Die Fluid-Messausgangsöffnungen am Gehäuse des Y-Stücks und die Fluid-Messeingangsöffnungen am Gehäuse des Elektronikmoduls können so zur Herstellung durchgängiger Strömungskanäle in verbundenem Zustand von Y-Stück und Elektronikmodul deckungsgleich übereinander angeordnet sein, wobei z. B. an einer oder beiden Gehäuseaußenseiten angeordnete Dichtungselemente eine gasdichte Verbindung ermöglichen.

Es kann auch vorgesehen sein, dass die Fluidmessausgänge des Y-Stücks als Nadeln ausgeführt sind, wobei die Fluidmesseingänge des Elektronikmoduls jeweils von einem durchstechbaren Dichtungselement, vorzugsweise einer sogenannten "selbstheilenden" Membran (z. B. einer Silikonmembran), verschlossen sind. Das Verbinden der beiden Bauteile geschieht hierbei mittels Durchstechens der nadelspitzen Fluidmessausgänge durch die Dichtungselemente. Indem "selbstheilende" Membranen als Dichtungselemente eingesetzt sind, werden die Fluidmesseingänge des Elektronikmoduls nach Entfernen des Y-Stücks durch die intrinsisch ablaufende "Reparatur" der Dichtungselemente wieder abgedichtet.

In gleicher Weise können auch die Fluidmessausgänge am Y-Stück mit einem Dichtungselement in Form einer "selbstheilenden" Membran verschlossen sein und die Fluidmesseingänge des Elektronikmoduls röhrenförmig ausgebildet sein bzw. Nadelspitzen aufweisen, die für eine Verbindung der beiden Bauteile durch die Membranen zu stechen sind.

Weiterhin ist vorgesehen, dass das Elektronikmodul mittels eines (mehradrigen) Kabels elektrisch mit dem Beatmungsgerät verbunden ist. Über dieses Kabel können die Stromversorgung für z. B. die Sensoren und die Auswerteeinheit sichergestellt sein sowie z. B. die Messsignale des Druck- und der Differenzdrucksensoren oder Signale der Auswerteeinheit zwischen dem Elektronikmodul und dem Beatmungsgerät übertragen werden. Es ist jedoch auch möglich, alle Signalleitungen elektrisch zu koppeln und die Signale über eine Busverbindung zum Beatmungsgerät zu führen.

Gemäß einer weiteren Ausgestaltung weist das Elektronikmodul einen fest mit seinem Gehäuse verbundenen Steckverbinder auf, wobei eine Verbindung zum Beatmungsschlauchsystem mittels eines mehradrigen Kabels hergestellt wird. Bei dieser Ausgestaltung wird das mehradrige Kabel des Beatmungsschlauchsystems zum Anbinden sowohl der Stromzuführung für das Elektronikmodul als auch der Sensoren des Elektronikmoduls an z.B. das Beatmungsgerät verwendet.

Das Y-Stück weist an seinem Patientenanschluss und den beiden Endbereichen am Inspirations- bzw. Exspirationszweig dem Fachmann bekannte Normanschlüsse nach DIN-EN-ISO 5356-1 auf.

Der Vorteil der erfindungsgemäßen Anordnung zur Messung des Volumenstromes bei der maschinellen Beatmung ist, dass der technische Totraum zwischen Y-Stück und Patient nahezu vollständig entfällt. Insbesondere bei Säuglingen, deren Atemvolumen z. B. 5 ml beträgt, ist das Entfallen des typischerweise etwa 2 ml großen technischen Totraums im zwischen Y-Stück und Tubuskonnektor angeordneten pneumatischen Widerstandselement von überlebenswichtiger Bedeutung.

Die Bestimmung des vom Patienten eingeatmeten bzw. ausgeatmeten Gasvolumens ergibt sich aus der Differenz der im Inspirationszweig und im Exspirationszweig gemessenen Gasflussmengen.

Außerdem wird für die Anordnung lediglich eine elektrische Zuleitung benötigt; lange pneumatische Verbindungsschläuche zum Beatmungsgerät, wie aus dem Stand der Technik bekannt, entfallen, sodass sowohl eine weniger umständliche Handhabung beim Anlegen des Beatmungsschlauches bzw. der Messanordnung als auch eine verbesserte Regelung der Beatmung ermöglicht ist.

Die Anordnung kann weiterhin eine Heizung mit einem Heizelement, z. B. in Form einer elektrischen Heizfolie oder eines andersgearteten Heizelementes, umfassen, die beispielsweise an einer Gehäuseaußenseite des Elektronikmoduls und in Kontakt zu dem Y-Stück bzw. dessen Widerstandselementen positioniert ist. Diese Heizung verhindert, dass sich in den pneumatischen Widerstandselementen und den Drucksensoren sowie in den Strömungskanälen Kondenswasser bildet.

Das Heizelement kann beispielsweise in Form eines beheizbaren Bandes ausgeführt sein, welches um das Y-Stück wickelbar und z. B. mittels Klettverschluss befestigbar ist. Das Heizelement kann auch in das Gehäuse des Elektronikmoduls integriert sein, wobei das Elektronikmodulgehäuse komplementär zum Y-Stück geformt ist (das Elektronikmodul weist z. B. dem Y-Stück formangepasste Einbuchtungen auf), d. h. in verbundenem Zustand von Elektronikmodul und Y-Stück ist eine formschlüssige Verbindung ausgebildet.

Um die Temperatur des Heizelementes den Erfordernissen entsprechend regeln, beispielsweise den Umgebungsbedingungen anpassen, zu können, sind in dem Elektronikmodul weiterhin ein Heizungs-Steuergerät mit einem Temperaturregelkreis sowie Mittel zur Bestimmung der Temperatur des Heizelementes selbst und/oder von vorgegebenen Punkten oder flächigen Bereichen an der Trennstelle zwischen Elektronikmodul und Y-Stück vorgesehen.

Da bei medizinische Anwendungen die Sensoren, die für die Regelung verwendet werden, nicht auch gleichzeitig der Überwachung der geregelten Größe dienen dürfen, sind zwei Mittel zur Bestimmung der Temperatur, z. B. Temperatursensoren mit entsprechenden Signalleitungen oder andere, gleichwertige Mittel für die Temperaturmessung, vorgesehen.

Vorzugsweise erfolgt die Temperaturmessung in einem elektrisch betriebenen Heizelement durch Bestimmung des temperaturabhängigen elektrischen Widerstandes des Heizelementes selbst und seine Einbeziehung in den Temperaturregelkreis des Heizungs-Steuergeräts, wie er dem Fachmann bekannt ist.

Das Heizungs-Steuergerät kann zum Beispiel in Form einer kleinen Leiterplatte, die (neben dem Druck- und den Differenzdrucksensoren) im Elektronikmodul angeordnet ist, realisiert sein. Ebenso kann das Heizungs-Steuergerät in die Auswerteeinheit des Elektronikmoduls integriert sein, d. h. das Heizungs-Steuergerät ist ein integraler Bestandteil der Auswerteeinheit.

Es kann auch vorgesehen sein, dass die Regelung der Temperatur in dem, elektrisch mit der Anordnung zur Messung des Volumenstromes verbundenem, Beatmungsgerät erfolgt, wobei der von dem Mittel zur Temperaturmessung (z. B. elektrisch) gemessene Temperaturwert an das Beatmungsgerät über Kabel weitergeleitet wird.

Die freien Drahtenden des die Signalleitungen und die elektrischen Zuleitungen für die Stromzuführung umfassenden Kabels können auf verschiedene Weise mit einem oder mehreren, beispielsweise je einen für die Signalleitungen und die Stromzuführung, Steckverbindern elektrisch verbunden sein. Gemäß einer Ausführungsform sind der Strom-Steckverbinder und der Sensor-Steckverbinder integral miteinander als ein einziger Steckverbinder ausgebildet, d. h. die freien Drahtenden des mehradrigen Kabels münden in einen einzigen gemeinsamen Steckverbinder. Indem ein mehradriges Kabel mit lediglich einem einzigen Steckverbinder zum Anbinden sowohl der Stromzuführung als auch der Sensoren an z. B. das Beatmungsgerät verwendet wird, kann der für das medizinische Personal beim Anschließen der Sensorvorrichtung anfallende Aufwand klein gehalten werden.

Es ist aber auch möglich, einen ersten Steckverbinder für die Drahtenden der Stromzuführung und weitere Steckverbinder für die Signalleitungen zu verwenden, wobei z. B. die Leitungen für Drucksignale an einen zweiten Steckverbinder, die Leitungen für Temperatursignale an einen dritten Steckverbinder und ggf. die Leitungen für den Temperaturregelkreis an einen vierten Steckverbinder angeschlossen sind.

Gemäß einer Ausgestaltungsvariante umfasst das Y-Stück zusätzlich mindestens einen Sensor zum Erfassen einer Temperatur und/oder Feuchtigkeit des Atemgases. Besagter Sensor weist ein Sensorteil bzw. einen Sensorabschnitt zum Erfassen der jeweiligen Messgrößen und Erzeugen entsprechender Messsignale sowie Signalleitungen zum Übertragen und Abgreifen der Messsignale auf.

Für eine Weiterleitung der Messsignale an das Elektronikmodul kann dieses einen Steckverbinder aufweisen, in welchen Steckverbinder von Signalleitungen der in dem Y-Stück untergebrachten Sensoren einsteckbar sind.

Es kann vorgesehen sein, dass das Y-Stück einen fest mit seinem Gehäuse verbundenen, zu dem (ebenfalls fest mit dessen Gehäuse verbundenen) Steckverbinder des Elektronikmoduls komplementären Steckverbinder (d. h. das Gegenstück) aufweist, wobei beide Steckverbinder bei Verbinden von Y-Stück und Elektronikmodul zwangsgeführt ineinandergreifen, d. h. eine elektrische Verbindung hergestellt wird, sodass ein zusätzliches manuelles Verbinden von Kabeln entfällt.

Gemäß einer weiteren Ausgestaltung weist das Y-Stück ein mit einem Datenkabel von Temperatursensoren des Inspirations-und/oder Exspirationszweiges verbindbares Elektrosteckersystem auf, wobei in verbundenem Zustand von Y-Stück und Elektronikmodul das Elektrosteckersystem in den Steckverbinder des Elektronikmoduls greift, sodass auch die von den Temperatursensoren der Beatmungsschläuche übermittelten Signale an das Elektronikmodul weiterleitbar sind.

Die mittels eines Temperatursensors erfasste Temperatur des durch das Y-Stück strömenden Atemgases kann z. B. zur Ausbildung eines Temperaturregelkreises an das Heizungs-Steuergerät übertragen werden. Mit der Verbesserung der Sensoren für die direkte Feuchtigkeitsmessung ist es auch möglich, den Temperaturregelkreis durch einen Feuchtigkeitsregelkreis zu ersetzen.

Bei Ausführung des Sensors als Feuchtigkeitssensor kann somit vorgesehen sein, die von dem Sensor erfassten Messwerte zur Ausbildung eines Feuchtigkeitsregelkreises bzw. feuchtigkeitsgeführten Regelkreises an das Heizungs-Steuergerät zu übertragen.

Gemäß einer Ausführung weist das Y-Stück mehrere Sensoren zur Bestimmung einer Temperatur und einer Feuchtigkeit des Atemgases auf. Insbesondere kann vorgesehen sein, einen ersten Sensor als Feuchtigkeitssensor auszubilden und die von demselben erfassten Feuchtigkeitsmesswerte z. B. zum Ausbilden eines feuchtigkeitsgeführten Regelkreises zum Regeln der Heizleistung zu verwenden und einen zweiten Sensor als Temperatursensor auszubilden und die von demselben erfassten Temperaturmesswerte z. B. zum Überwachen der patientennahen Atemgastemperatur zu verwenden.

Die Sensoren können mittels entsprechender Ausgestaltung des Y-Stücks derart in dem Y-Stück angeordnet sein, dass sie in den Atemgasstrom hineinragen, sodass mittels eines als Temperatursensor ausgebildeten Sensors die Temperatur des durch den Gaskanal des Y-Stücks strömenden Atemgases und mittels eines als Feuchtigkeitssensor ausgebildeten Sensors die Feuchtigkeit des durch den Gaskanal strömenden Atemgases erfasst werden kann.

Es kann insbesondere vorgesehen sein, dass die mehreren Sensoren bezüglich einer Längsachse des Gaskanals im Y-Stück zueinander winkelversetzt angeordnet sind.

Indem die Sensoren bezüglich der Längsachse des Gaskanals des Y-Stücks zueinander winkelversetzt angeordnet sind, kann verhindert werden, dass einer der Sensoren im Strömungsschatten bzw. Windschatten des jeweils anderen Sensors angeordnet ist.

Die Erfindung wird nachfolgend anhand eines Ausführungsbeispiels mit Bezug auf die Figuren veranschaulicht, wobei gleiche oder ähnliche Merkmale mit gleichen Bezugszeichen versehen sind. Hierzu zeigen in schematischer Darstellung die
- Figur 1:: die Anordnung in perspektivischer Sicht; und
- Figur 2: einen Längsschnitt entlang eines Schenkels des Y-Stücks.

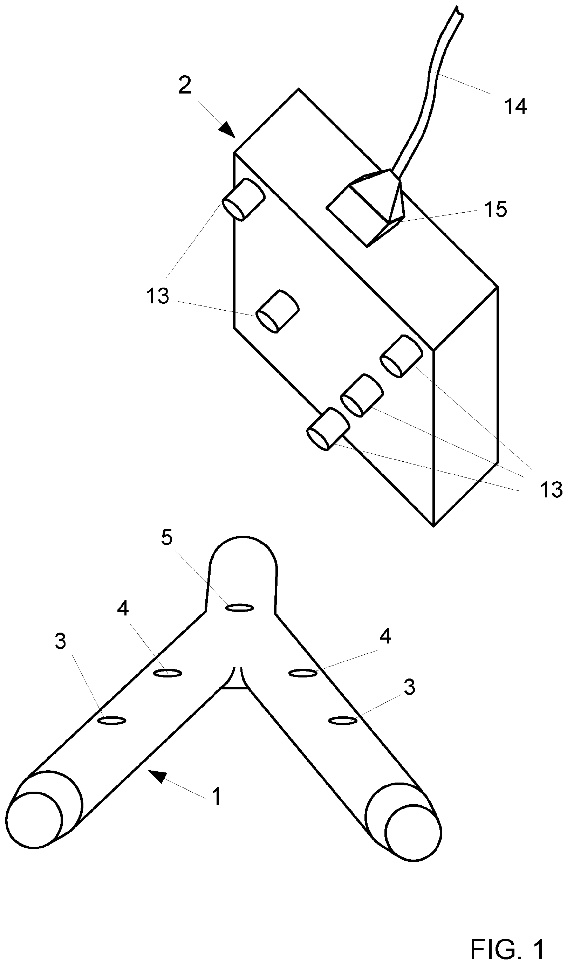

Die Figur 1 zeigt das Y-Stück 1 und das Elektronikmodul 2 in getrenntem Zustand.

Das Y-Stück 1 weist die Fluidmessausgänge 3, 4 und 5 auf, in welche die röhrenförmigen Fluidmesseingänge 13 des Elektronikmoduls 2 einsteckbar sind.

Das Elektronikmodul 2 weist außerdem den Elektrosteckverbinder 15 zum Anschluss des Kabels 14 zwecks Verbinden mit dem Beatmungsgerät auf.

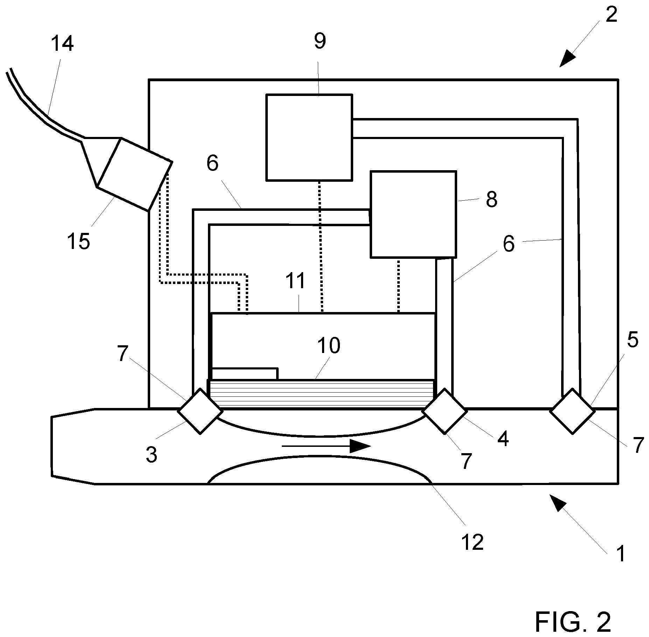

Im schematischen Längsschnitt gemäß Figur 2 ist ein pneumatisches Widerstandselement 12 des Y-Stücks 1 zu sehen, an dessen beiden Endbereichen der patientenferne Fluidmessausgang 3 und der patientennahe Fluidmessausgang 4 angeordnet sind, die jeweils die Keimsperreinrichtung 7 aufweisen.

Das Elektronikmodul 2 umfasst den Differenzdrucksensor 8 und den Drucksensor 9, die über die pneumatischen Verbindungsleitungen 6 mit den Fluidmessausgängen 3, 4, 5 des Y-Stücks 1 verbunden sind. Außerdem umfasst das Elektronikmodul 2 die Auswerteeinheit 11 und das Heizelement 10, welches in Kontakt zum Y-Stück 1 angeordnet ist.

Die Stromzuführung und die Signalleitungen der Auswerteeinheit 11 (bzw. der Sensoren 8 und 9) münden in den Elektrosteckverbinder 15, der diese durch eine Durchgangsöffnung im Gehäuse des Elektronikmoduls 2 hindurchgeführt. In den Elektrosteckverbinder 15 ist das mehradrige Kabel 14 eingesteckt.

### Liste der verwendeten Bezugszeichen

- 1: Y-Stück
- 2: Elektronikmodul
- 3: patientenferner Fluidmessausgang
- 4: patientennaher Fluidmessausgang
- 5: Fluidmessausgang
- 6: pneumatische Verbindungsleitung / Strömungskanal
- 7: Keimsperreinrichtung
- 8: Differenzdrucksensor
- 9: Drucksensor
- 10: Heizvorrichtung / Heizsystem
- 11: Auswerteeinheit
- 12: pneumatisches Widerstandselement
- 13: Fluidmesseingang
- 14: Kabel
- 15: Elektrosteckverbinder für Signalleitungen und Stromzufuhr

## Patentansprüche

1. Anordnung zur Messung des Volumenstromes eines Patienten bei der maschinellen Beatmung, aufweisend:
- ein Y-Stück (1) zum Verbinden eines Inspirationszweigs von einer Beatmungsmaschine sowie eines Exspirationszweiges mit einem Tubuskonnektor;
- ein erstes pneumatisches Widerstandselement (12) im Inspirationszweig und ein zweites pneumatisches Widerstandselement (12) im Exspirationszweig sowie
- einen Differenzdrucksensor (8),
**dadurch gekennzeichnet, dass**
- das erste pneumatische Widerstandselement (12) und das zweite pneumatische Widerstandselement (12) monolithisch in das Y-Stück (1) integriert sind;
- das Y-Stück (1) für jedes seiner monolithisch integrierten pneumatischen Widerstandselemente (12) einen mit demselben in fluidischer Verbindung stehenden patientennahen Fluidmessausgang (4) und patientenfernen Fluidmessausgang (3) und zusätzlich einen zentral angeordneten fünften Fluidmessausgang (5) beinhaltet, die jeweils eine Keimsperreinrichtung (7) aufweisen;
- das Y-Stück (1) als Einweg-Einheit konzipiert ist;
- die Anordnung ein separates Elektronikmodul (2) aufweist, welches den Differenzdrucksensor (8), einen zweiten Differenzdrucksensor (8), einen Drucksensor (9), ein mit dem Drucksensor (9) und den Differenzdrucksensoren (8) verbundene Auswerteeinheit (11) sowie pneumatische Verbindungsleitungen (6) umfasst, wobei die pneumatischen Verbindungsleitungen (6) den Drucksensor (9) und die Differenzdrucksensoren (8) jeweils mit in einem Gehäuse des Elektronikmoduls (2) eingebrachten Fluidmesseingängen (13) verbinden;
- das Elektronikmodul (2) lösbar mit dem Y-Stück (1) verbindbar ist, wobei in verbundenem Zustand eine gasdichte Verbindung zwischen den Fluidmesseingängen (13) des Elektronikmoduls (2) und den Fluidmessausgängen (3, 4, 5) des Y-Stückes (1) gebildet ist; und
- das Elektronikmodul (2) als Mehrweg-Einheit konzipiert ist.

2. Anordnung nach Anspruch 1, **dadurch gekennzeichnet, dass** das Elektronikmodul (2) ein Heizelement (10), das in thermischem Kontakt zu dem mit dem Elektronikmodul (2) verbundenen Y-Stück (1) ist, und Mittel zur Bestimmung der Temperatur des Heizelements (10) und/oder des Y-Stücks (1) aufweist, wobei das Heizelement (10) eine an dem Gehäuse des Elektronikmoduls (2) angeordnete Heizfolie ist oder eine solche umfasst.

3. Anordnung nach einem der vorstehenden Ansprüche, **dadurch gekennzeichnet, dass** das Elektronikmodul (2) ein Heizungs-Steuergerät mit einem Temperaturregelkreis aufweist, das elektrisch mit dem Heizelement (10) und den Mitteln zur Bestimmung der Temperatur verbunden ist.

4. Anordnung nach Anspruch 3, **dadurch gekennzeichnet, dass** das Heizungs-Steuergerät in der Auswerteeinheit (11) integriert ist.

5. Anordnung nach einem der vorstehenden Ansprüche, **dadurch gekennzeichnet, dass** die Fluidmessausgänge (3, 4, 5) auf dem Y-Stück (1) in Form eines spiegelsymmetrischen oder asymmetrischen Fünfecks angeordnet sind.

6. Anordnung nach einem der vorstehenden Ansprüche, **dadurch gekennzeichnet, dass** das Elektronikmodul (2) einen Elektrosteckverbinder (15) für ein mehradriges Kabel aufweist, an welchen Zuleitungen zur Stromzuführung für das Elektronikmodul (2) als auch Signalleitungen für Signale der Auswerteeinheit (11) an das Beatmungsschlauchsystem und/oder das Beatmungsgerät angeschlossen sind.

7. Anordnung nach einem der vorstehenden Ansprüche, **dadurch gekennzeichnet, dass** die Fluidmessausgänge (3, 4, 5) als mit einer selbstheilenden Membran verschlossene Bohrungen durch die Gehäusewand des Y-Stücks (1) und die Fluidmesseingänge (13) des Elektronikmoduls (2) jeweils als rohrförmige Stutzen ausgebildet sind, wobei in verbundenem Zustand von Elektronikmodul (2) und Y-Stück (1) die Stutzen der Fluidmesseingänge (13) des Elektronikmoduls (2) durch die Membranen der Fluidmessausgänge (3, 4, 5) des Y-Stücks (1) geführt sind.

8. Anordnung nach einem der vorstehenden Ansprüche, **dadurch gekennzeichnet, dass** das Y-Stück (1) auf seinem Gehäuse eine Nut aufweist und das Elektronikmodul (2) auf seinem Gehäuse einen formschlüssig in die Nut des Y-Stückes (1) einführbaren Steg aufweist, wobei die Fluidmesseingänge (13) des Elektronikmoduls (2) in verbundenem Zustand von Elektronikmodul (2) und Y-Stück (1) deckungsgleich zu den Fluidmessausgängen (3, 4, 5) des Y-Stücks (1) angeordnet sind und mittels Dichtungen gasdichte Strömungskanäle gebildet sind.

9. Anordnung nach einem der vorstehenden Ansprüche, **dadurch gekennzeichnet, dass** das Gehäuse des Elektronikmoduls (2) aus einem antibakteriellem Material besteht oder antibakteriell beschichtet ist.

10. Anordnung nach einem der vorstehenden Ansprüche, **dadurch gekennzeichnet, dass** die Auswerteeinheit (11) als Microcontroller oder als ASIC ausgeführt ist.

11. Anordnung nach einem der vorstehenden Ansprüche, **dadurch gekennzeichnet, dass** die Auswerteeinheit (11) eingerichtet ist, eine Bestimmung eines Volumenstromes von und zu dem beatmeten Patienten mittels Auswertung der von den beiden Differenzdrucksensoren (8) erfassten Differenzdrücke durchzuführen.

## Claims

1. Assembly for measuring the flow rate of patient during mechanical ventilation, having:
- a Y-shaped piece (1) for connecting an inspiration branch from a ventilation machine and an expiration branch to a tube connector;
- a first pneumatic resistance element (12) in the inspiration branch and a second pneumatic resistance element (12) in the expiration branch, and
- a differential pressure sensor (8),
**characterised in that**
- the first pneumatic resistance element (12) and the second pneumatic resistance element (12) are monolithically integrated into the Y-shaped piece (1);
- the Y-shaped piece (1) contains, for each of its monolithically integrated pneumatic resistance elements (12), a fluid measurement outlet (4) near the patient and a fluid measurement outlet (3) remote from the patient, which are in fluidic connection with said Y-shaped piece, and additionally a centrally arranged fifth fluid measurement outlet (5), which each have a germ barrier device (7);
- the Y-shaped piece (1) is designed as a disposable unit;
- the assembly has a separate electronics module (2) which comprises the differential pressure sensor (8), a second differential pressure sensor (8), a pressure sensor (9), an evaluation unit (11) connected to the pressure sensor (9) and the differential pressure sensors (8), and pneumatic connecting lines (6), wherein the pneumatic connecting lines (6) connect the pressure sensor (9) and the differential pressure sensors (8) in each case to fluid measurement inlets (13) incorporated in a housing of the electronics module (2);
- the electronics module (2) is detachably connectable to the Y-shaped piece (1), wherein in the connected state a gas-tight connection is formed between the fluid measurement inlets (13) of the electronics module (2) and the fluid measurement outlets (3, 4, 5) of the Y-shaped piece (1); and
- the electronics module (2) is designed as a reusable unit.

2. Assembly according to claim 1, **characterised in that** the electronics module (2) has a heating element (10) which is in thermal contact with the Y-shaped piece (1) connected to the electronics module (2), and means for determining the temperature of the heating element (10) and/or of the Y-shaped piece (1), wherein the heating element (10) is or comprises a heating foil arranged on the housing of the electronics module (2).

3. Assembly according to any of the preceding claims, **characterised in that** the electronics module (2) has a heating control device with a temperature control circuit which is electrically connected to the heating element (10) and the means for determining the temperature.

4. Assembly according to claim 3, **characterised in that** the heating control device is integrated into the evaluation unit (11).

5. Assembly according to any of the preceding claims, **characterised in that** the fluid measurement outlets (3, 4, 5) are arranged on the Y-shaped piece (1) in the form of a mirror-symmetrical or asymmetrical pentagon.

6. Assembly according to any of the preceding claims, **characterised in that** the electronics module (2) has an electrical plug connector (15) for a multicore cable, to which supply lines for the power supply for the electronics module (2) as well as signal lines for signals of the evaluation unit (11) are connected to the ventilation tube system and/or the ventilation device.

7. Assembly according to any of the preceding claims, **characterised in that** the fluid measurement outlets (3, 4, 5) are formed as bores through the housing wall of the Y-shaped piece (1) which are closed by a self-healing membrane and the fluid measurement inlets (13) of the electronics module (2) are each formed as tubular sockets, wherein, when the electronics module (2) and Y-shaped piece (1) are connected, the sockets of the fluid measurement inlets (13) of the electronics module (2) are guided through the membranes of the fluid measurement outlets (3, 4, 5) of the Y-shaped piece (1).

8. Assembly according to any of the preceding claims, **characterised in that** the Y-shaped piece (1) has a groove on its housing and the electronics module (2) has on its housing a web which can be inserted into the groove of the Y-shaped piece (1) in a form-fitting manner, wherein the fluid measurement inlets (13) of the electronics module (2) are arranged congruently with the fluid measurement outlets (3, 4, 5) of the Y-shaped piece (1) when the electronics module (2) and Y-shaped piece (1) are connected, and gas-tight flow channels are formed by means of seals.

9. Assembly according to any of the preceding claims, **characterised in that** the housing of the electronics module (2) consists of an antibacterial material or is antibacterially coated.

10. Assembly according to any of the preceding claims, **characterised in that** the evaluation unit (11) is designed as a microcontroller or as an ASIC.

11. Assembly according to any of the preceding claims, **characterised in that** the evaluation unit (11) is configured to determine a flow rate from and to the ventilated patient by evaluating the differential pressures detected by the differential pressure sensors (8).

## Revendications

1. Ensemble pour mesurer le débit volumique d'un patient sous ventilation artificielle, présentant :
- une pièce en Y(1) pour relier une conduite d'inspiration d'une ventilation mécanique assistée ainsi qu'une conduite d'expiration à un connecteur de tube ;
- un premier élément de résistance (12) pneumatique dans la conduite d'inspiration et un deuxième élément de résistance (12) pneumatique dans la conduite d'expiration ainsi
- qu'un capteur de pression différentielle (8),
**caractérisé en ce que**
- le premier élément de résistance (12) pneumatique et le deuxième élément de résistance (12) pneumatique sont intégrés de manière monolithique dans la pièce en Y (1) ;
- la pièce en Y(1) contient, pour chacun de ses éléments de résistance (12) pneumatiques intégrés de manière monolithique, une sortie de mesure de fluide (4) proche du patient se trouvant en communication fluidique avec lui et une sortie de mesure de fluide (3) éloignée du patient et, en supplément, une cinquième sortie de mesure de fluide (5) disposée de manière centrale, qui présentent respectivement un dispositif de blocage anti-germes (7) ;
- la pièce en Y (1) est conçue en tant qu'une unité à usage unique ;
- l'ensemble présente un module électronique (2) séparé, lequel comprend le capteur de pression différentielle (8), un deuxième capteur de pression différentielle (8), un capteur de pression (9), une unité d'évaluation (11) reliée au capteur de pression (9) et aux capteurs de pression différentielle (8) ainsi que des conduits de liaison pneumatiques (6), dans lequel les conduits de liaison pneumatiques (6) relient le capteur de pression (9) et les capteurs de pression différentielle (8) respectivement à des entrées de mesure de fluide (13) introduites dans un boîtier du module électronique (2) ;
- le module électronique (2) peut être relié de manière amovible à la pièce en Y (1), dans lequel dans un état relié, une liaison étanche aux gaz est formée entre les entrées de mesure de fluide (13) du module électronique (2) et les sorties de mesure de fluide (3, 4, 5) de la pièce en Y (1) ; et
- le module électronique (2) est conçu en tant qu'une unité à usage multiple.

2. Ensemble selon la revendication 1, **caractérisé en ce que** le module électronique (2) présente un élément de chauffage (10), qui est en contact thermique avec la pièce en Y (1) reliée au module électronique (2), et des moyens pour définir la température de l'élément de chauffage (10) et/ou de la pièce en Y (1), dans lequel l'élément de chauffage (10) est un film de chauffage disposé sur le boîtier du module électronique (2) ou comprend un film de ce type.

3. Ensemble selon l'une quelconque des revendications précédentes, **caractérisé en ce que** le module électronique (2) présente un appareil de commande de chauffage avec un circuit de régulation de température, qui est relié de manière électrique à l'élément de chauffage (10) et aux moyens pour définir la température.

4. Ensemble selon la revendication 3, **caractérisé en ce que** l'appareil de commande de chauffage est intégré dans l'unité d'évaluation (11).

5. Ensemble selon l'une quelconque des revendications précédentes, **caractérisé en ce que** les sorties de mesure de fluide (3, 4, 5) sont disposées sur la pièce en Y (1) sous la forme d'un pentagone à symétrie en miroir ou asymétrique.

6. Ensemble selon l'une quelconque des revendications précédentes, **caractérisé en ce que** le module électronique (2) présente un connecteur enfichable électrique (15) pour un câble à brins multiples, par lequel des conduits d'arrivée pour amener du courant pour le module électronique (2) ainsi que des câbles de signalisation pour des signaux de l'unité d'évaluation (11) sont raccordés au système tubulaire de ventilation et/ou à l'appareil de ventilation.

7. Ensemble selon l'une quelconque des revendications précédentes, **caractérisé en ce que** les sorties de mesure de fluide (3, 4, 5) sont réalisées en tant qu'alésages fermés avec une membrane auto-réparatrice à travers la paroi de boîtier de la pièce en Y (1) et les entrées de mesure de fluide (13) du module électronique (2) sont réalisées respectivement en tant que tubulures tubulaires, dans lequel dans un état relié du module électronique (2) et de la pièce en Y (1), les tubulures des entrées de mesure de fluide (13) du module électronique (2) sont guidées à travers les membranes des sorties de mesure de fluide (3, 4, 5) de la pièce en Y (1).

8. Ensemble selon l'une quelconque des revendications précédentes, **caractérisé en ce que** la pièce en Y (1) présente sur son boîtier une rainure et le module électronique (2) présente sur son boîtier une entretoise pouvant être introduite par complémentarité de forme dans la rainure de la pièce en Y (1), dans lequel les entrées de mesure de fluide (13) du module électronique (2) sont disposées, dans un état relié de module électronique (2) et de pièce en Y (1), de manière superposée par rapport aux sorties de mesure de fluide (3, 4, 5) de la pièce en Y (1) et des canaux d'écoulement étanches au gaz sont formés au moyen de joints d'étanchéité.

9. Ensemble selon l'une quelconque des revendications précédentes, **caractérisé en ce que** le boîtier du module électronique (2) est constitué d'un matériau antibactérien ou est doté d'un revêtement antibactérien.

10. Ensemble selon l'une quelconque des revendications précédentes, **caractérisé en ce que** l'unité d'évaluation (11) est réalisée en tant que microcontrôleur ou en tant qu'ASIC [application-specific integrated circuit - circuit intégré propre à une application].

11. Ensemble selon l'une quelconque des revendications précédentes, **caractérisé en ce que** l'unité d'évaluation (11) est mise au point pour mettre en œuvre une définition d'un débit volumique depuis et vers le patient sous assistance respiratoire au moyen de l'évaluation des pressions différentielles détectées par les deux capteurs de pression différentielle (8).
